# EUROPÄISCHE PATENTSCHRIFT

(11) **EP 1 499 582 B1**
(45) Veröffentlichungstag und Bekanntmachung des Hinweises auf die Patenterteilung: **15.12.2010**
(21) Anmeldenummer: 03722529.9
(22) Anmeldetag: 23.04.2003
(51) Int. Cl.: C07C 217/54, C07C 213/08

(54) **VERFAHREN ZUR CHLORIERUNG TERTIÄRER ALKOHOLE**
METHOD FOR CHLORINATING TERTIARY ALCOHOLS
PROCEDE POUR CHLORURER DES ALCOOLS TERTIAIRES

(30) Priorität: 26.04.2002 DE 10218862
(43) Veröffentlichungstag der Anmeldung: 26.01.2005
(73) Patentinhaber: Grünenthal GmbH, 52078 Aachen (DE)
(72) Erfinder: BUSCHMANN, Helmut, E-08950 Esplugues de Llobregat (ES); HELL, Wolfgang, 52066 Aachen (DE); KEGEL, Markus, 52078 Aachen (DE)
(74) Vertreter: Brosch, Oliver
(86) Internationale Anmeldenummer: PCT/EP2003/004213
(87) Internationale Veröffentlichungsnummer: WO 2003/091199

(56) Entgegenhaltungen:
- EP-A- 0 753 506

## Beschreibung

Die vorliegende Erfindung betrifft ein Verfahren zur Umwandlung einer tertiären OH-Gruppe einer organischen Verbindung in eine tertiäre Cl-Gruppe der organischen Verbindung.

Insbesondere betrifft die Erfindung ein Verfahren zur Herstellung einer Verbindung der allgemeinen Formel (I) 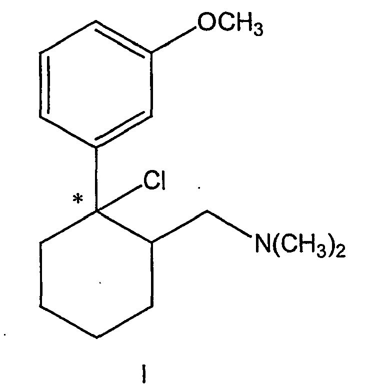 und zwar in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in dargestellter Form oder in Form ihrer Salze, insbesondere der physiologisch verträglichen Salze, besonders bevorzugt in Form des Hydrochlorids, oder in Form ihrer Solvate, insbesondere der Hydrate.

Wie aus der EP 0 753 506 A1 bekannt, ist die Verbindung der Formel (I) analgetisch wirksam und dient ferner als Zwischenprodukt zur Herstellung weiterer analgetisch wirksamer Substanzen. Die EP 0 753 506 A1 offenbart ein Verfahren zur Herstellung der Verbindung der Formel (I) ausgehend von einer Verbindung der allgemeinen Formel (II) 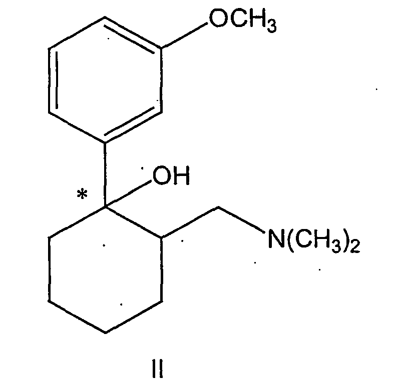 deren Herstellung literaturbekannt ist (K. Flick et al., Arzneim.-Forsch./Drug Res. 28 (1), Heft 1a (1978)), bei dem zur Umwandlung der tertiären Alkoholfunktion am Cyclohexylrest in eine Cl-Substitution unter Retention am mit* gekennzeichneten C-Atom Thinoylchlorid als Chlorierungsreagenz und zugleich als Lösungsmittel verwendet wird. Dieses Verfahren erfordert demnach große Mengen an Thionylchlorid. Ferner erweist sich die anschließende Aufarbeitung unter Austreiben des überschüssigen Thionylchlorids mit einem Stickstoffgasstrom oder mittels Destillation als belastend für Mensch, Umwelt und Arbeitsausrüstung. Außerdem werden nur schlechte Ausbeuten (maximal 55 % der Theorie) erzielt.

Es ist daher eine Aufgabe der vorliegenden Erfindung, ein Verfahren bereitzustellen, mit dem eine organische Verbindung mit einer tertiären OH-Gruppe, insbesondere eine Verbindung der Formel (II), zu einer entsprechenden organischen Verbindung mit einer tertiären Cl-Gruppe, insbesondere zu einer Verbindung der Formel (I), in hoher Ausbeute umgewandelt wird. Ferner ist es wünschenswert, durch dieses Verfahren die Menge des einzusetzenden Chlorierungsmittels im Vergleich zum literaturbekannten Verfahren zu verringern.

Die Aufgabe wird gelöst durch ein Verfahren zur Umwandlung einer tertiären OH-Gruppe einer organischen Verbindung in eine tertiäre Cl-Gruppe der organischen Verbindung, das dadurch gekennzeichnet ist, daß der tertiäre Alkohol in einem Lösungsmittel, das aus der Gruppe ausgewählt ist, die Toluol, o-Xylol, m-Xylol, p-Xylol und Gemische davon umfaßt, suspendiert beziehungsweise gelöst, die entstehende Suspension beziehungsweise Lösung mit Thionylchlorid versetzt und dann die entstehende organische Verbindung mit der tertiären Cl-Gruppe von den weiteren Reaktionsbestandteilen abgetrennt wird.

Bevorzugt ist es dabei, mindestens 1 Äquivalent und nicht mehr als 3 Äquivalente Thionylchlorid einzusetzen. Bevorzugtes Lösungsmittel ist Toluol.

Die Abtrennung der entstehenden organischen Verbindung mit der tertiären Cl-Gruppe kann auf unterschiedliche Art und Weise erfolgen. So kann die organische Verbindung mit der tertiären Cl-Gruppe nach erfolgter Umsetzung durch Abdestillieren der weiteren Reaktionsbestandteile mit dem Lösungsmittel isoliert werden. Dabei ist unter Abdestillieren auch das teilweise Entfernen des Lösungsmittels mit den weiteren Reaktionsbestandteilen unter Wärmezufuhr im Vakuum zu verstehen. Es ist auch möglich, die organische Verbindung mit der tertiären Cl-Gruppe nach erfolgter Umsetzung durch Abkühlen unter Raumtemperatur als Feststoff zu erhalten und sie dann bei Temperaturen zwischen 35°C und 75°C, vorzugsweise im Vakuum, zu trocknen. Bevorzugt wird die halogenierte organische Verbindung in Form ihres Hydrochlorids erhalten.

Dieses erfindungsgemäße Verfahren erlaubt die Synthese organischer Verbindungen mit einer tertiären Cl-Gruppe ausgehend von dem korrespondierenden tertiären Alkohol unter Erhalt der Stereochemie am stereogenen Zentrum in hohen Ausbeuten von über 70% und unter Reduktion der einzusetzenden Thionylchlorid-Menge.

Als besonders vorteilhaft hat sich das erfindungsgemäße Verfahren zur Herstellung einer Verbindung der Formel (I) 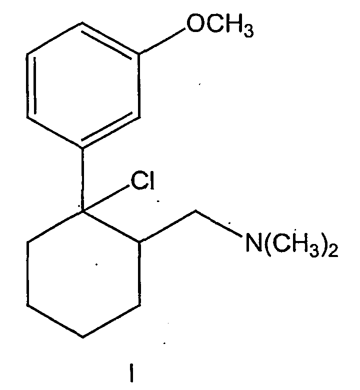 in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in dargestellter Form oder in Form ihrer Salze, insbesondere der physiologisch verträglichen Salze, besonders bevorzugt in Form des Hydrochlorids, und/oder in Form ihrer Solvate, insbesondere der Hydrate; erwiesen, wobei das Verfahren dadurch gekennzeichnet ist , daß eine Verbindung der Formel (II) 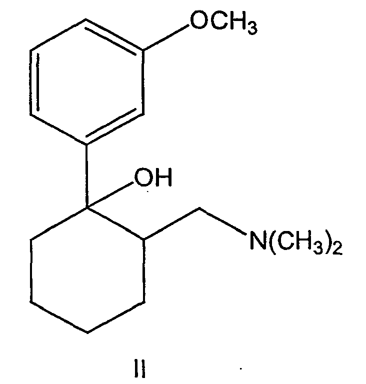 in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in dargestellter Form oder in Form ihrer Salze, insbesondere der physiologisch verträglichen Salze, besonders bevorzugt in Form des Hydrochlorids, und/oder in Form ihrer Solvate, insbesondere der Hydrate;
(a) in einem Lösungsmittel, das aus der Gruppe ausgewählt ist, die Toluol, o-Xylol, m-Xylol, p-Xylol und Gemische davon umfaßt, suspendiert wird;
(b) die entstehende Suspension mit Thionylchlorid versetzt wird; und
(c) das Reaktionsprodukt mit der tertiären Cl-Gruppe der Formel (I) von den weiteren Reaktionsbestandteilen abgetrennt wird.

Es ist dabei vorteilhaft, daß die Abtrennung (c) des Reaktionsprodukts der Formel (I) durch Abdestillieren des Lösungsmittels mit den weiteren Reaktionsbestandteilen erfolgt. Dabei ist unter Abdestillieren auch das teilweise Entfernen des Lösungsmittels mit den weiteren Reaktionsbestandteilen unter Wärmezufuhr im Vakuum zu verstehen. Alternativ kann die Abtrennung (c) durch Ausfällen des Reaktionsprodukts der Formel (I) mittels Abkühlen des Reaktionsgemischs unter Raumtemperatur durchgeführt und das Reaktionsprodukt anschließend bei Temperaturen zwischen 35°C und 75°C, vorzugsweise im Vakuum, getrocknet werden.

Als Lösungsmittel beziehungsweise Lösungsvermittler ist Toluol bevorzugt. Die Verwendung anderer unpolarer Lösungsmittel als Toluol und/oder Xylol(e), zum Beispiel THF, Heptan, Hexan, Cyclohexan, haben sich als nicht vorteilhaft erwiesen, weil dann die Umsetzung unter Bildung zahlreicher Nebenprodukte, d.h. nicht ausreichend selektiv abläuft. Ferner ist es bevorzugt, 1 bis 2 Äquivalente, insbesondere 1,5 bis 1,7 Äquivalente, besonders bevorzugt 1,6 Äquivalente Thionylchlorid - bezogen auf die Verbindung der Formel (II) - einzusetzen. Dies stellt eine drastische Verminderung der einzusetzenden Menge an Thionylchlorid gegenüber dem aus der EP 0 753 506 A1 bekannten Verfahren dar. Auch werden bei vollständigem Umsatz der Ausgangsverbindung (II) deutlich höhere Ausbeuten erzielt, die über 70% der Theorie liegen.

Die Umsetzung der Verbindung (II) erfolgt bevorzugt bei erhöhter Temperatur, insbesondere bei einer Temperatur von 30 bis 50°C bevorzugt 35 bis 45 °C, für 1 bis 4 h, bevorzugt 2 bis 3 h, wobei die Reaktionszeit auch darüber oder darunter liegen kann.

Es hat sich gezeigt, daß die erfindungsgemäße Umsetzung von (II) zu (I) in Gegenwart von Katalysatoren, zum Beispiel Dimethylformamid, weder höhere Umsatzraten noch bessere Ausbeuten ergibt; allerdings stören diese Katalysatoren auch nicht den Verlauf der Reaktion.

Zumeist wird die Ausgangsverbindung (II) in Form ihres Hydrochlorids eingesetzt. Sie wird in dem inerten und unpolaren Lösungsvermittler Toluol suspendiert, in dem sie sich weder in der Kälte noch in der Wärme löst. Die Zugabe des seinerseits relativ unpolaren Thionylchlorids sollte daran nichts ändern, jedoch beobachtet man im Laufe der Reaktion, daß sich das Edukt (11) vollständig auflöst. Ferner ist es überraschend und für die gesamte Prozeßführung sehr vorteilhaft, daß sich - anders als bei Umsetzungen mit Thionylchlorid, wie z.B. in dem literaturbekannten Verfahren der EP 0 753 506 A1, üblich - kein Schwefeldioxid-Gas und kein Chlorwasserstoff-Gas beim Erwärmen entwickelt. Es ist möglich, daß das Edukt der Formel (II) und/oder das Produkt der Formel (I) einen Komplex mit Schwefeldioxid und Chlorwasserstoff eingehen, so daß die Verbindungen (I) und (II) in Lösung bleiben und SO₂ und HCl nicht als Gase freigesetzt werden. Die Komplexbildung von Aminen mit SO₂ in organischen Lösungsmitteln ist in der Literatur bekannt (siehe zum Beispiel J. Grundnes, S. D. Christian, J. Am. Chem. Soc. (1968) 90, 2239-2245), während die - hypothetische - Komplexbildung mit HCl bislang nicht beobachtet wurde.

Unabhängig davon, ob die Hypothese der Komplexbildung zutrifft, erweist sich der überraschende Umstand, daß die Reaktanten der Umsetzung in homogener Lösung verbleiben, als vorteilhaft im Hinblick auf Ausbeute und Umsetzung. Ein weiterer Vorteil besteht darin, daß es möglich ist, das gewünschte Reaktionsprodukt dadurch vom Lösungsvermittler und den weiteren Reaktionsbestandteilen abzutrennen, daß der Lösungsvermittler, bevorzugt Toluol, abdestilliert wird, vorzugsweise im Vakuum; dabei werden zugleich das gebildete Schwefeldioxid und der Chlorwasserstoff entfernt.

Ebenso vorteilhaft ist es, die Abtrennung des Produkts (I) durch Abkühlung des Reaktionsgemischs auf eine Temperatur unterhalb der Raumtemperatur, bevorzugt auf 0 bis 10°C, insbesondere auf etwa 2° bis 4°C, zu erreichen, was zum Ausfällen des Rohprodukts führt. Anschließende Behandlung im Trockenschrank bei erhöhten Temperaturen von vorzugsweise 35 bis 75°C, insbesondere 40°C, und bevorzugt im Vakuum, insbesondere bei einem Druck von 1 bis 300 mbar, bevorzugt 50 bis 180 mbar, ganz besonders bevorzugt 50 bis 150 mbar, liefert das Reinprodukt (I), vorzugsweise als Hydrochlorid.

Es ist besonders bevorzugt, das erfindungsgemäße Verfahren mit den Stereoisomeren der Verbindung (II) auszuführen, die die Konfiguration (IIa) und/oder (IIb) aufweisen:

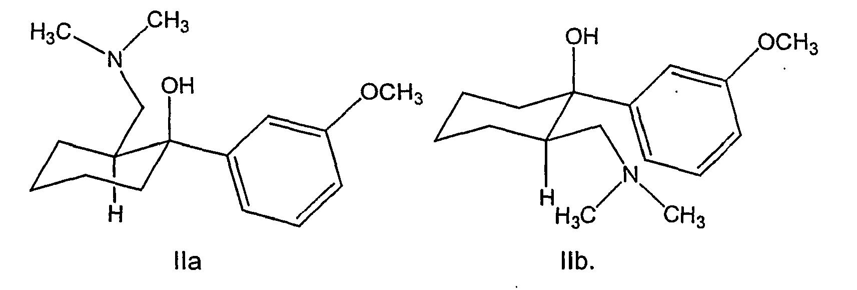

Die erfindungsgemäße Umsetzung mit Thionylchlorid führt - erwartungsgemäß unter Erhalt (Retention) der Stereochemie am Reaktionszentrum - zu den entsprechenden Stereoisomeren der Verbindung (I). Weiter bevorzugt ist es, die Verbindung (II) in dem erfindungsgemäßen Verfahren als Enantiomer mit der absoluten Konfiguration (IIa), d.h. als (1S,2S)-2-Dimethylaminomethyl-1-(3-methoxyphenyl)-cyclohexanol, insbesondere als Hydrochlorid, einzusetzen. Als Produkt wird dabei (1S,2S)-[2-Chlor-2-(3-methoxyphenyl)-cyclohexylmethyl]-dimethylamin, insbesondere in Form des HCl-Salzes, erhalten.

Wie aus der EP 753 506 A1 bekannt, sind die Verbindungen der Formel (I) analgetisch wirksam und können als Zwischenverbindungen zur Herstellung weiterer analgetisch wirksamer Cyclohexyl-Verbindungen eingesetzt werden.

Es ist daher ebenfalls bevorzugt, eine nach dem erfindungsgemäßen Verfahren hergestellte Verbindung der Formel (I) durch (d) katalytische Hydrierung in eine Verbindung der Formel (III) überzuführen:

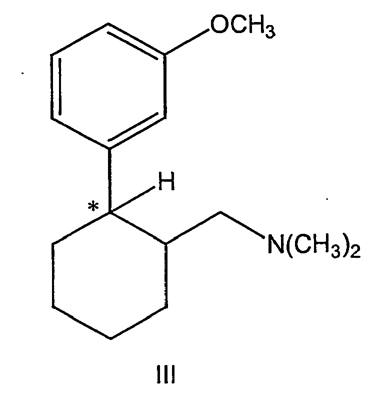

Vorzugsweise erfolgt die Hydrierung durch heterogene Katalyse mit Palladium auf Aktivkohle, dessen Menge in einem weiten Bereich zwischen 1 % über 5 % bis 10 % Palladium auf Aktivkohle variiert werden kann. Geeignete Lösungsmittel sind Alkylalkohole, insbesondere Methanol und Ethanol. Üblicherweise wird die Verbindung (I) als Hydrochlorid eingesetzt. Bevorzugt wird der Hydrierungsschritt (d) unter Retention der Stereochemie an dem mit einem Stern (*) gekennzeichneten C-Atom in Formel (I) bzw. (III) ausgeführt. Die gegebenenfalls gebildete Menge an Verbindung (III) mit invertierter Stereochemie am C*-Atom ist derart gering, daß sie ohne weiteres bei der üblichen Aufarbeitung abgetrennt werden kann. Somit eignet sich dieses Verfahren hervorragend, um ausgehend von dem Enantiomer mit der absoluten Konfiguration (IIa) über das entsprechende Enantiomer der Verbindung (Ia) 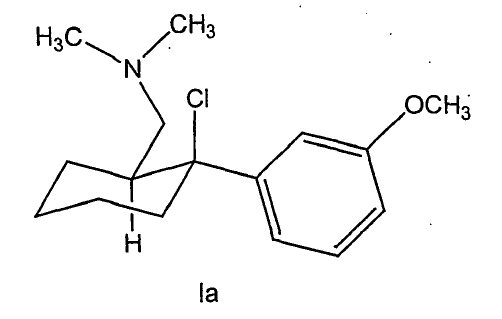 die Verbindung (III) als Enantiomer mit der absoluten Konfiguration (IIIa) 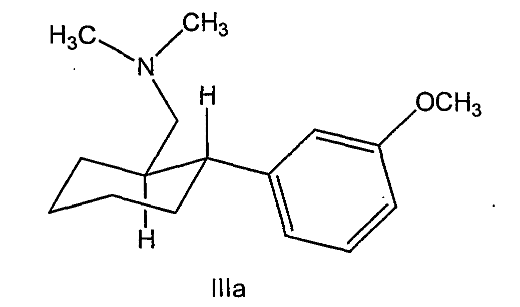 herzustellen. (Entsprechend kann ausgehend vom optischen Antipoden (IIb) über (Ib) die zu (IIIa) spiegelbildliche Verbindung (IIIb) hergestellt werden.)

Alternativ zu der Hydrierung mittels heterogener Katalyse ist die Verbindung der Formel (III) auch durch Hydrierung mit komplexen Zinkborhydriden aus (I) zugänglich, wie dies in EP 0 753 506 A1 beschrieben ist.

Ferner kann die Verbindung der Formel (III), vorzugsweise nach dem erfindungsgemäßen Verfahren hergestellt, in einem weiteren Schritt (e) mittels Methyletherspaltung in die Verbindung der Formel (IV) überführt werden:

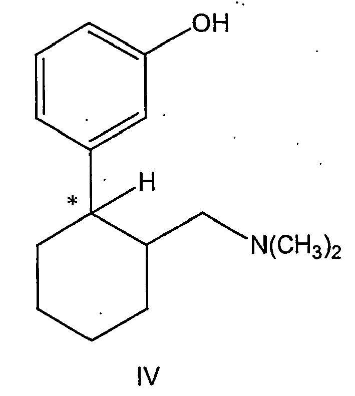

Für diese Methyletherspaltung wurde bislang wäßrige BromwasserstoffSäure verwendet (EP 0 753 506 A1). Diese Reaktionsführung hat jedoch verschiedene Nachteile. So muß zur Erzielung befriedigender Ausbeuten ein hoher Überschuß der Säure eingesetzt werden, der nach erfolgter Umsetzung aufwendig abdestilliert und vernichtet werden muß. Besonders gravierend ist die Tatsache, daß bei der Verwendung von HBr als Reaktionsprodukt Methylbromid anfällt; Methylbromid ist ein giftiges, leicht entzündliches Gas mit einem Siedepunkt von 4°C, das vermutlich die Ozonschicht der Erdatmosphäre schädigt und für das daher in der Europäischen Union ein Produktionsverbot erwogen wird.

Es ist daher ein Verfahren erforderlich, das die gewünschte Methyletherspaltung unter Vermeidung des Einsatzes von HBr ermöglicht und zugleich hohe Ausbeuten des Produkts (IV) garantiert.

Es wurde nun gefunden, daß durch die gleichzeitige Verwendung von Methionin und Methansulfonsäure die Methyletherspaltung von (III) zu (IV) außerordentlich leicht und in sehr hohen Ausbeuten von über 70 % ausgeführt werden kann. (Die Verwendung von Methionin/Methansulfonsäure zur Herstellung von Phenolen wurde von N. Fujii et al., J. Chem. Soc. Perkin I (1977) 2288-2298, beschrieben, die jedoch keine aminsubstituierten Verbindungen umsetzten.) Dabei wird die Verbindung (III), bevorzugt in Form des Hydrochlorids, in einem Gemisch aus einem großen Überschuß an Methansulfonsäure, üblicherweise zwischen 5 und 40 Äquivalente, insbesondere 10 bis 30 Äquivalente, besonders bevorzugt etwa 20 Äquivalente,und 1 bis 2 Äquivalenten, bevorzugt etwa 1,1 bis 1,3 Äquivalenten Methionin suspendiert und dann für 1 bis 12 h, bevorzugt 3 bis 8 h, insbesondere 5 h erwärmt auf Temperaturen von 50 bis 100°C, bevorzugt 70 bis 90°C. Nach Abkühlen und übliche Aufarbeitung wird das gewünschte Phenol (IV) in hohen Ausbeuten ohne Nebenprodukte und allenfalls geringsten Mengen an nicht umgesetztem Ausgangsmaterial (III) erhalten, bevorzugt als Hydrochlorid; durch Umkristallisation aus Wasser wird (IV) in Form von Hydrochlorid-Hydraten erhalten. Es ist dabei überraschend, daß sich die ansonsten im sauren Medium recht labile Dimethylamino-Gruppe gegenüber der in großem Überschuß vorhandenen Methansulfonsäure als stabil erweist.

Somit steht nunmehr ein effizientes Verfahren zur Herstellung der aus der EP 0 753 506 A1 als analgetisch bekannten Verbindung mit der absoluten Konfiguration (IVa) ausgehend von dem tertiären Alkohol (II) mit der absoluten Konfiguration (IIa) zur Verfügung, das auch im technischen Maßstab durchgeführt werden kann. Die Synthesesequenz ist in Schema 1 wiedergegeben. (Entsprechendes gilt für den optischen Antipoden von (IVa)) mit (1S,2S)-Konfiguration).

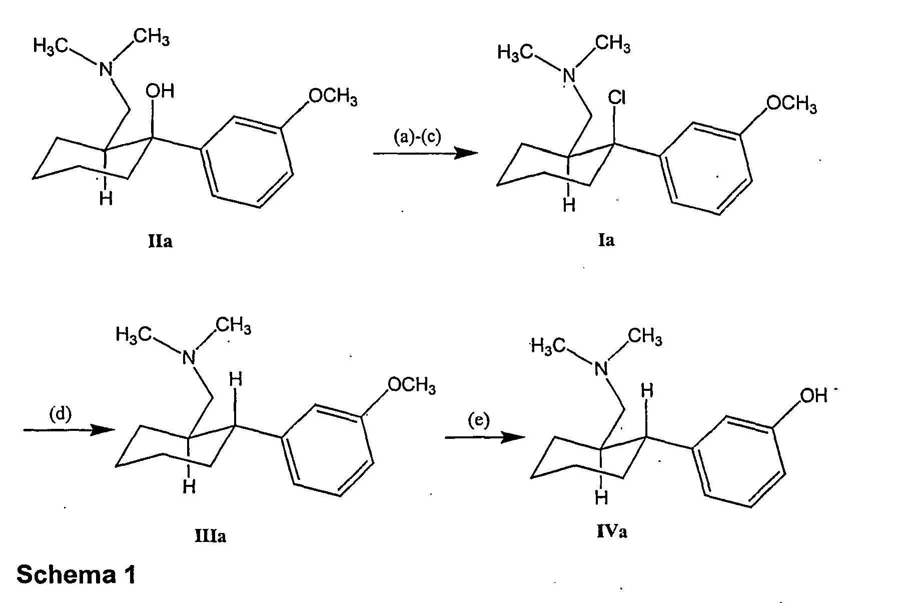

Die Erfindung wird nachfolgend durch Beispiele veranschaulicht, ohne sie darauf zu beschränken.

### Beispiele

Die Synthesen wurden mit handelsüblichen Reagenzien und Substanzen oder mit Verbindungen, die nach literaturbekannten Verfahren hergestellt wurden, durchgeführt.

Die Identifizierung der Reaktionsprodukte und die Bestimmung der chemischen und der optischen Reinheit erfolgte mittels NMR-Spektroskopie und HPLC-Chromatögraphie.

### Beispiel 1

### Chlorierung mit Thionylchlorid: Herstellung von (1S,2S)-[2-Chlor-2-(3-methoxy-phenyl)-cyclohexylmethyl]-dimethyl-aminhydrochlorid

3 kg (10 mol) (1S,2S)-2-Dimethylaminomethyl-1-(3-methoxy-phenyl)-cyclohexanolhydrochlorid wurden in 10 l Toluol suspendiert und auf 30°C erwärmt. Innerhalb von 10 min wurden 1,9 kg (16 mol) Thionylchlorid zugetropft. Die Mischung wurde auf 35° - 45°C erwärmt. Nach 2 - 3 h war eine klare Lösung entstanden. Die weitere Verarbeitung erfolgte nach Variante A beziehungsweise B.

### Variante A:

Es wurden 3 kg Toluol im Vakuum über einen Gaswäscher abdestilliert. Es entwichen Chlorwasserstoffgas und Schwefeldioxid mit dem Abdestillieren des Toluols. Danach wurde auf 2°C abgekühlt und 2 h bei dieser Temperatur gerührt. Der ausgefallene Niederschlag wurde abzentrifugiert und gründlich mit Toluol nachgewaschen. Das Produkt konnte direkt oder nach Trocknung für die nachfolgende Hydrierung eingesetzt werden. Die Ausbeute betrug 2,87 kg (90 % d. Th.) (1 S,2S)-[2-Chlor-2-(3-methoxyphenyl)-cyclohexylmethyl]-dimethylaminhydrochlorid.

### Variante B:

Die Reaktionsmischung wurde auf 2°C gekühlt und 4 h gerührt. Der gebildete Niederschlag wurde abzentrifugiert und im Trockenschrank bei 40°C und einem Vakuum von < 150 mbar 17 h getrocknet. Das so erhaltene Produkt konnte für die nachfolgende Hydrierung eingesetzt werden. Die Ausbeute betrug 2,55 kg (80 % d. Th.) (1 S,2S)-[2-Chlor-2-(3-methoxy-phenyl)-cyclohexylmethyl]-dimethylaminhydrochlorid.

Die Identifizierung erfolgte mittels Vergleich der analytischen Daten des in Variante A beziehungsweise B erhaltenen Produkts und der aus der EP 0 753 506 A1 bekannten Verbindung, der Identität zeigte. Die Bestimmung der chemischen und optischen Reinheit erfolgte durch HPLC an einer Nucleosil 100-5 C8 HD-Säule (250 x 3 mm) mit Gradienten-Elution mit Acetonitril/Wasser; die Detektion erfolgte UV-spektrometrisch bei 210 nm.

### Beispiel 2

### Heterogen katalysierte Hydrierung: Hydrierung von (1S,2S)-[2-Chlor-2-(3-methoxy-phenyl)-cyclohexylmethyl]-dimethylaminhydrochlorid

3,18 kg (10 mol) (1S,2S)-[2-Chlor-2-(3-methoxy-phenyl)-cyclohexylmethyl] dimethylaminhydrochlorid (aus Beispiel 1) wurden in 15 l Methanol gelöst, und 350 g Palladium auf Aktivkohle 5 % wurden hinzugegeben. Bei Normaldruck und Raumtemperatur wurde solange Wasserstoff eingeleitet, bis die Hydrierung vollständig abgelaufen war. Der Katalysator wurde abgesaugt, das Lösungsmittel eingeengt und die Base mit wäßriger Natriumhydroxidlösung freigesetzt. Nach Ausschütteln der wäßrigen Phase mit Essigsäureethylester und Abdestillieren des organischen Lösungsmittels verblieben 2,47 kg (100 % d. Th.) (1R,2R)-[2-(3-Methoxyphenyl)-cyclohexylmethyl]-dimethylamin als leicht gelb gefärbtes Öl.

Das Öl wurde in Aceton gelöst, und mit Salzsäuregas wurde das Hydrochlorid gefällt. Man erhielt 2,27 kg (80 % d. Th.) (1 R,2R)-[2-(3-Methoxy-phenyl)-cyclohexylmethyl]-dimethylaminhydrochlorid als farbloses Pulver mit einem Gehalt von < 10 % an (1 R,2S)-[2-(3-Methoxy-phenyl)-cyclohexylmethyl]-dimethylaminhydrochlorid, das durch Umkristallisieren aus 2-Propanol entfernt wurde.

Die Identifizierung erfolgte mittels Vergleich der analytischen Daten des erhaltenen Produkts und der aus der EP 0 753 506 A1 bekannten Verbindung, der Identität zeigte. Die Bestimmung der chemischen und optischen Reinheit erfolgte durch HPLC an einer Nucleosil 100-5 C8 HD-Säule (250 x 3 mm) mit Gradienten-Elution mit Acetonitril/Wasser; die Detektion erfolgte UV-spektrometrisch bei 210 nm.

### Beispiel 3

### Methyletherspaltung mit Methansulfonsäure/Methionin: Herstellung von (1R,2R)-3-(2-Dimethylaminomethyl-cyclohexyl)-phenolhydrochlorid

2,83 kg (10 mol) (1R,2R)-[2-(3-Methoxyphenyl)-cyclohexy-methyl]-dimethylaminhydrochlorid wurden in einer Mischung aus 8,70 l Methansulfonsäure und 1,50 kg (D,L)-Methionin suspendiert und für 5 h auf 70° - 90°C erwärmt. Es wurde auf 30°C gekühlt und mit 32 %iger wäßriger Natronlauge ein pH-Wert von 12 -14 eingestellt. Die Base wurde mit Essigsäureethylester extrahiert. Nach Einengen des organischen Lösungsmittel und der Fällung in Aceton mit Salzsäuregas (oder wäßriger Salzsäure) verblieben 1,86 kg (80 %) (1R,2R)-3-(2-Dimethylaminomethyl-cyclohexyl)-phenolhydrochlorid mit einem Gehalt von < 1 % des Ausgangsproduktes (1R,2R)-[2-(Methoxyphenyl)-cyclohexyl-methyl]-dimethylaminhydrochlorid.

Umkristallisation aus Wasser erbrachte farblose Kristalle (1,5 kg, 80 %) des (1R,2R)-3-(2-Dimethylaminomethyl-cyclohexyl)-phenolhydrochloriddihydrats.

Die Identifizierung erfolgte mittels Vergleich der analytischen Daten des erhaltenen Produkts und der aus der EP 0 753 506 A1 bekannten Verbindung, der Identität zeigte. Die Bestimmung der chemischen und optischen Reinheit erfolgte durch HPLC an einer Nucleosil 100-5 C8 HD-Säule (250 x 3 mm) mit Gradienten-Elution mit Acetonitril/Wasser; die Detektion erfolgte UV-spektrometrisch bei 210 nm.

## Patentansprüche

1. Verfahren zur Umwandlung einer tertiären OH-Gruppe einer organischen Verbindung in eine tertiäre Cl-Gruppe der organischen Verbindung, **dadurch gekennzeichnet, daß** der tertiäre Alkohol in einem Lösungsmittel, das aus der Gruppe ausgewählt ist, die Toluol, o-Xylol, m-Xylol, p-Xylol und Gemische davon umfaßt, suspendiert beziehungsweise gelöst, die entstehende Suspension beziehungsweise Lösung mit Thionylchlorid versetzt und dann die entstehende organische Verbindung mit der tertiären Cl-Gruppe von den weiteren Reaktionsbestandteilen abgetrennt wird.

2. Verfahren nach Anspruch 1, **dadurch gekennzeichnet, daß** die organische Verbindung mit der tertiären Cl-Gruppe nach erfolgter Umsetzung durch Abdestillleren der weiteren Reaktionsbestandtelle mit dem Lösungsmittel erhalten wird.

3. Verfahren nach Anspruch 1, **dadurch gekennzeichnet, daß** die organische Verbindung mit der tertiären Cl-Gruppe nach erfolgter Umsetzung durch Abkühlen unter Raumtemperatur als Feststoff erhalten und bei Temperaturen zwischen 35°C und 75°C, vorzugsweise im Vakuum, getrocknet wird.

4. Verfahren zur Herstellung einer organischen Verbindung mit einer tertiären Cl-Gruppe nach Anspruch 1, **dadurch gekennzeichnet, dass**
die organische Verbindung mit einer tertiären Cl-Gruppe eine Verbindung der allgemeinen Formel (I) ist 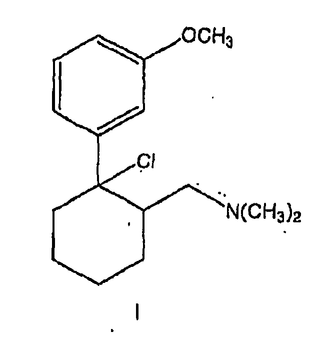 In Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
In dargestellter Form oder in Form ihrer Salze, insbesondere der physiologisch verträglichen Salze, besonders bevorzugt in Form des Hydrochlorids, und/oder in Form ihrer Solvate, insbesondere der Hydrate;
der tertiäre Alkohol eine Verbindung der allgemeinen Formel (II) ist 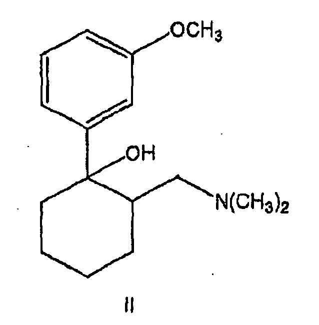 In Form ihrer Racemate, ihrer reinen Stereoisomeren, insbesondere Enantiomeren oder Diastereomeren, oder In Form von Mischungen der Stereoisomeren, insbesondere der Enantiomeren oder Diastereomeren, In einem beliebigen Mischungsverhältnis;
In dargestellter Form oder In Form ihrer Salze, Insbesondere der physiologisch verträglichen Salze, besonders bevorzugt in Form des Hydrochlorids, und/oder in Form ihrer Solvate, insbesondere der Hyd rate;
und die Verbindung der allgemeinen Formel (II)
(a) in einem Lösungsmittel, das aus der Gruppe ausgewählt ist, die Toluol, o-Xylol, m-Xylol, p-Xylol und Gemische davon umfaßt, suspendiert wird;
(b) die entstehende Suspension mit Thionylchlorid versetzt wird; und
(c) das Reaktionsprodukt mit der tertiären Cl-Gruppe der Formel (i) von den weiteren Reaktionsbestandteilen abgetrennt wird.

5. Verfahren nach Anspruch 4, **dadurch gekennzeichnet, daß** die Abtrennung (c) des Reaktionsprodukts der Formel (I) durch Abdestillieren des Lösungsmittels mit den weiteren Reaktionsbestandtellen erfolgt.

6. Verfahren nach Anspruch 4, **dadurch gekennzeichnet, daß** die Abtrennung (c) durch Ausfällen des Reaktionsprodukts der Formel (i) mittels Abkühlen des Reaktionsgemischs unter Raumtemperatur erfolgt und das Reaktionsprodukt bei Temperaturen zwischen 35°C und 75°C, vorzugsweise im Vakuum, getrocknet wird.

7. Verfahren nach Irgendeinem der Ansprüche 1 bis 6, **dadurch gekennzeichnet, daß** das Lösungsmittel Toluol ist.

8. Verfahren nach irgendeinem der Ansprüche 1 bis 7, **dadurch gekennzeichnet, daß** 1 bis 2 Äquivalente, insbesondere 1,5 bis 1,7 Äquivalente, besonders bevorzugt 1,6 Äquivalente Thionylchlorid - bezogen auf die Verbindung der Formel (II) - eingesetzt werden.

9. Verfahren nach irgendeinem der Ansprüche 1 bis 8, **dadurch gekennzeichnet, daß** die Umsetzung mit Thionylchlorid bei einer Temperatur von 30 bis 50°C, bevorzugt 35 bis 45 °C, für 1 bis 4 h, bevorzugt 2 bis 3 h, erfolgt.

10. Verfahren nach irgendeinem der Ansprüche 4 bis 9, **dadurch gekennzeichnet, daß** die Verbindung (ii) die Konfiguration (IIa) und/oder (IIb) aufweist:

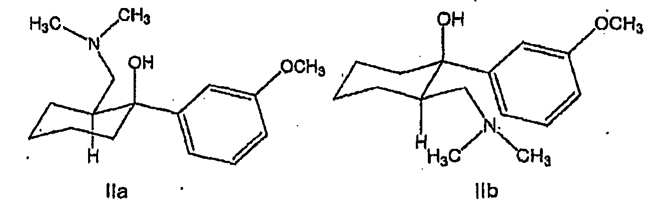

11. Verfahren nach irgeneinem der Ansprüche 4 bis 10; **dadurch gekennzeichnet, daß** die Verbindung (ii) als Enantiomer mit der absoluten Konfiguration (IIa) eingesetzt wird:

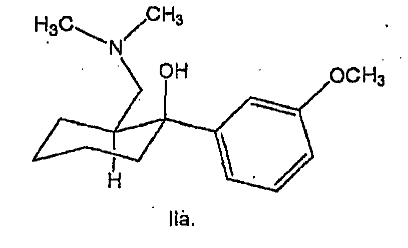

12. Verfahren nach irgendeinem der Ansprüche 4 bis 11
zur Herstellung einer Verbindung der allgemeinen Formel (III) 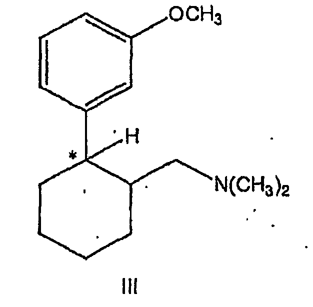 in Form Ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in dargestellter Form oder in Form ihrer Salze, insbesondere der physiologisch verträglichen Salze, besonders bevorzugt in Form des Hydrochlorids, und/oder in Form ihrer Solvate, insbesondere der Hydrate;
**dadurch gekennzeichnet, daß** die Verbindung der Formel (I) in einem Schritt
(d) einer katalytischen Hydrierung mit Palladium-auf-Aktivkohle als Katalysator unterworfen wird.

13. Verfahren nach Anspruch 12, **dadurch gekennzeichnet, daß** die Hydrierung (d) unter Retention der Stereochemie an dem mit einem Stern (*) **gekennzeichnet**en C-Atom in Formel (I) bzw. (III) erfolgt.

14. Verfahren nach irgendeinem der Ansprüche 12 oder 13
zur Herstellung einer Verbindung (IV) 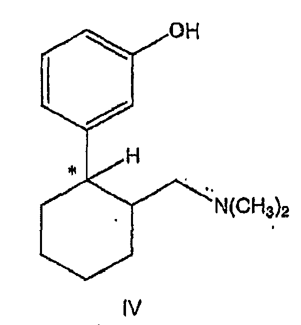 in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in dargestellter Form oder in Form ihrer Salze, insbesondere der physiologisch verträglichen Salze, besonders bevorzugt In Form des Hydrochlorids, oder In Form ihrer Solvate, insbesondere der Hydrate; **dadurch gekennzeichnet, daß** die Verbindung (III), in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in dargestellter Form oder in Form ihrer Salze, insbesondere der physiologisch verträglichen Salze, besonders bevorzugt in Form des Hydrochlorids, und/oder in Form ihrer Solvate, insbesondere der Hydrate;
in einem Schritt
(e) einer Methyletherspaltung mit Methionin/Methansulfonsäure unterworfen wird.

15. Verfahren nach Anspruch 14, **dadurch gekennzeichnet, daß** ausgehend von der Verbindung mit der absoluten Konfiguration (IIa) 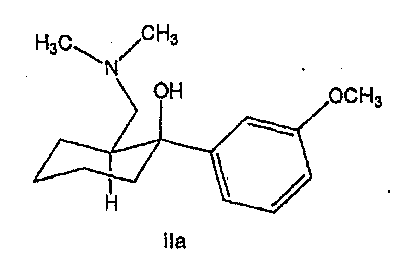 durch Ausführung der Verfahrensschritte (a) bis (e) die Verbindung mit der absoluten Konfiguration (IVa) 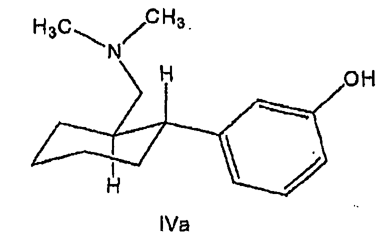 in Form ihres Hydrochlorids und/oder ihres Hydrochloriddihydrats hergestellt wird.

## Claims

1. Process for converting a tertiary hydroxyl group of an organic compound into a tertiary Cl group of the organic compound, **characterised in that** the tertiary alcohol is suspended or dissolved in a solvent selected from the group comprising toluene, o-xylene, m-xylene, p-xylene and mixtures thereof, thionyl chloride is added to the resultant suspension or solution and the resultant organic compound comprising the tertiary Cl group is then separated off from the further reaction components.

2. Process according to claim 1, **characterised in that** the organic compound comprising the tertiary Cl group is obtained after the reaction has taken place by distilling off the further reaction components with the solvent.

3. Process according to claim 1, **characterised in that** the organic compound comprising the tertiary Cl group is obtained as a solid after the reaction has taken place by cooling to below ambient temperature and is dried at temperatures between 35 °C and 75 °C, preferably in a vacuum.

4. Process for producing an organic compound comprising a tertiary Cl group according to claim 1, **characterised in that**
the organic compound comprising a tertiary Cl group is a compound of the general formula (I) 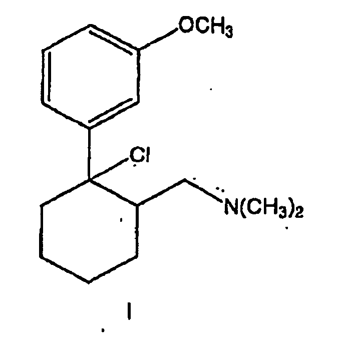 in the form of its racemates, its pure stereoisomers, in particular enantiomers or diastereomers, or in the form of mixtures of the stereoisomers, in particular of the enantiomers or diastereomers, in any mixing ratio;
in the illustrated form or in the form of its salts, in particular the physiologically acceptable salts, more preferably in the form of the hydrochlorides, and/or in the form of its solvates, in particular the hydrates;
the tertiary alcohol is a compound of the general formula (II) 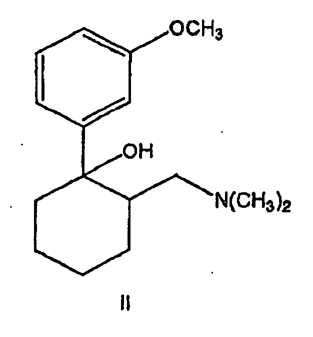 in the form of its racemates, its pure stereoisomers, in particular enantiomers or diastereomers, or in the form of mixtures of the stereoisomers, in particular of the enantiomers or diastereomers, in any mixing ratio;
in the illustrated form or in the form of its salts, in particular the physiologically acceptable salts, more preferably in the form of the hydrochlorides, and/or in the form of its solvates, in particular the hydrates;
and the compound of the general formula (II)
(a) is suspended in a solvent selected from the group comprising toluene, o-xylene, m-xylene, p-xylene and mixtures thereof;
(b) thionyl chloride is added to the resultant suspension; and
(c) the reaction product comprising the tertiary Cl group of formula (I) is separated off from the further reaction components.

5. Process according to claim 4, **characterised in that** the reaction product of formula (I) is separated off (c) by distilling off the solvent with the further reaction components.

6. Process according to claim 4, **characterised in that** the step of separating off (c) the reaction product of formula (I) by precipitation is carried out by cooling the reaction mixture to below ambient temperature and the reaction product is then dried at temperatures between 35 °C and 75 °C, preferably in a vacuum.

7. Process according to any one of claims 1 to 4, **characterised in that** the solvent is toluene.

8. Process according to any one of claims 1 to 7, **characterised in that** 1 to 2 equivalents, in particular 1.5 to 1.7 equivalents, more preferably 1.6 equivalents of thionyl chloride - based on the compound of formula (II) - are added.

9. Process according to any one of claims 1 to 8, **characterised in that** the reaction with thionyl chloride is carried out at a temperature of 30 to 50 °C, preferably 35 to 45 °C, for 1 to 4 h, preferably 2 to 3 h.

10. Process according to any one of claims 4 to 9, **characterised in that** the compound (II) has the configuration (IIa) and/or (IIb):

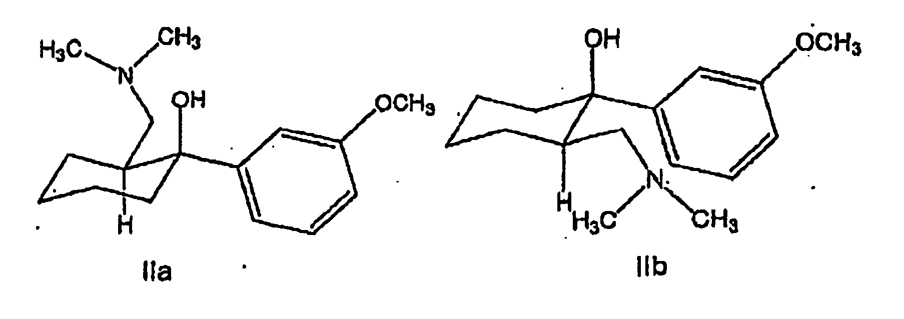

11. Process according to any one of claims 4 to 10, **characterised in that** the compound (II) is added as an enantiomer having the absolute configuration (IIa):

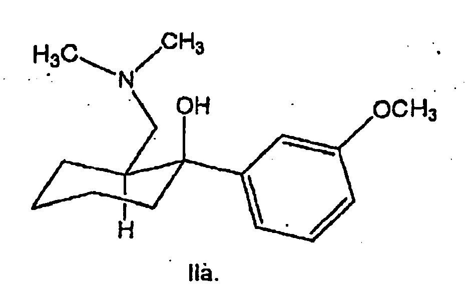

12. Process according to any one of claims 4 to 11 for producing a compound of the general formula (III) 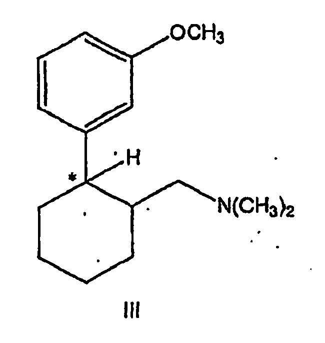 in the form of its racemates, its pure stereoisomers, in particular enantiomers or diastereomers, or in the form of mixtures of the stereoisomers, in particular of the enantiomers or diastereomers, in any mixing ratio;
in the illustrated form or in the form of its salts, in particular the physiologically acceptable salts, more preferably in the form of the hydrochlorides, and/or in the form of its solvates, in particular the hydrates;
**characterised in that** in a step
(d) the compound of the formula (I) is subjected to catalytic hydrogenation using palladium on activated carbon as a catalyst.

13. Process according to claim 12, **characterised in that** in the hydrogenation (d) is carried out while retaining the stereochemistry on the carbon atom marked by an asterisk (*) in formula (I) and formula (III).

14. Process according to either claim 12 or claim 13, for producing a compound (IV) 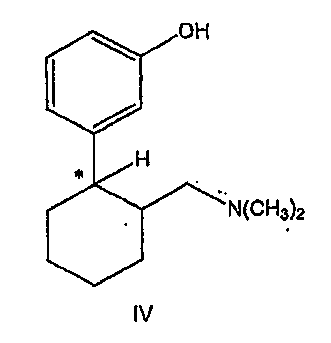 in the form of its racemates, its pure stereoisomers, in particular enantiomers or diastereomers, or in the form of mixtures of the stereoisomers, in particular of the enantiomers or diastereomers, in any mixing ratio;
in the illustrated form or in the form of its salts, in particular the physiologically acceptable salts, more preferably in the form of the hydrochlorides, or in the form of its solvates, in particular the hydrates;
**characterised in that** the compound (III), in the form of its racemates, its pure stereoisomers, in particular enantiomers or diastereomers, or in the form of mixtures of the stereoisomers, in particular of the enantiomers or diastereomers, in any mixing ratio;
in the illustrated form or in the form of its salts, in particular the physiologically acceptable salts, more preferably in the form of the hydrochlorides, and/or in the form of its solvates, in particular the hydrates;
in a step
(e) is subjected to methyl ether cleavage using methionine/methane sulphonic acid.

15. Process according to claim 14, **characterised in that**, starting from the compound having the absolute configuration (IIa) 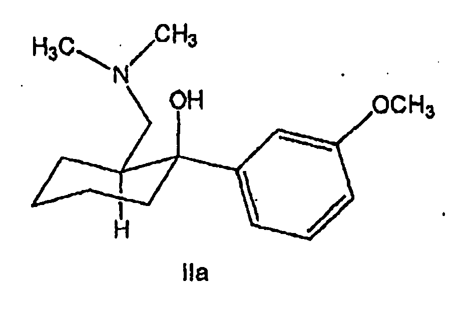 the process steps (a) to (e) are performed to produce a compound having the absolute configuration (IVa) 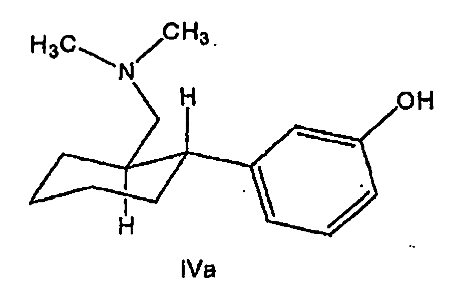 in the form of its hydrochloride and/or its hydrochloride hydrate.

## Revendications

1. Procédé pour la conversion d'un groupe OH tertiaire d'un composé organique en un groupe Cl tertiaire du composé organique, **caractérisé en ce qu'**on dissout ou met en suspension l'alcool tertiaire dans un solvant, qui est choisi dans le groupe comprenant le toluène, l'o-xylène, le m-xylène, le p-xylène et des mélanges de ceux-ci, on ajoute du chlorure de thionyle à la solution ou suspension résultante et ensuite on sépare le composé organique résultant, comportant le groupe Cl tertiaire, d'avec les autres composants du mélange réactionnel.

2. Procédé selon la revendication 1, **caractérisé en ce que** le composé organique comportant le groupe Cl tertiaire est obtenu, une fois la réaction effectuée, par élimination par distillation des autres composants du mélange réactionnel avec le solvant.

3. Procédé selon la revendication 1, **caractérisé en ce que** le composé organique comportant le groupe Cl tertiaire est obtenu sous forme de solide, une fois la réaction effectuée, par refroidissement jusqu'au-dessous de la température ambiante et séché à des températures comprises entre 35 °C et 75 °C, de préférence sous vide.

4. Procédé pour la préparation d'un composé organique comportant un groupe Cl tertiaire selon la revendication 1, **caractérisé en ce que**
le composé organique comportant un groupe Cl tertiaire est un composé de formule générale (I) 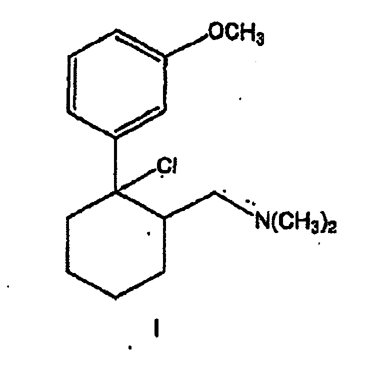 sous forme de ses racémates, de ses stéréoisomères purs, en particulier énantiomères ou diastéréoisomères, ou sous forme de mélanges des stéréoisomères, en particulier des énantiomères ou diastéréoisomères, en un rapport quelconque de mélange ;
sous la forme représentée ou sous forme de ses sels, en particulier des sels physiologiquement acceptables, de façon particulièrement préférée sous forme du chlorhydrate, et/ou sous forme de ses produits de solvatation, en particulier des hydrates ;
l'alcool tertiaire est un composé de formule générale (II) 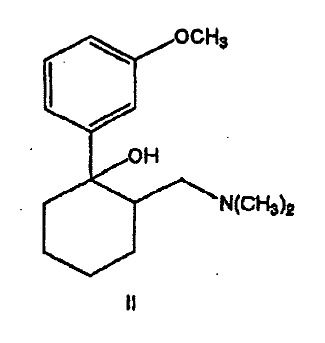 sous forme de ses racémates, de ses stéréoisomères purs, en particulier énantiomères ou diastéréoisomères, ou sous forme de mélanges des stéréoisomères, en particulier des énantiomères ou diastéréoisomères, en un rapport quelconque de mélange ;
sous la forme représentée ou sous forme de ses sels, en particulier des sels physiologiquement acceptables, de façon particulièrement préférée sous forme du chlorhydrate, et/ou sous forme de ses produits de solvatation, en particulier des hydrates ;
et le composé de formule générale (II)
(a) est mis en suspension dans un solvant, qui est choisi dans le groupe comprenant le toluène, l'o-xylène, le m-xylène, le p-xylène et des mélanges de ceux-ci ;
(b) on ajoute du chlorure de thionyle à la suspension résultante ; et
(c) on sépare le produit de réaction de formule (I), comportant le groupe Cl tertiaire, d'avec les autres composants du mélange réactionnel.

5. Procédé selon la revendication 4, **caractérisé en ce que** la séparation (c) du produit de réaction de formule (I) s'effectue par élimination par distillation du solvant avec les autres composants du mélange réactionnel.

6. Procédé selon la revendication 4, **caractérisé en ce que** la séparation (c) s'effectue par précipitation du produit de réaction de formule (I) par refroidissement du mélange réactionnel jusqu'au-dessous de la température ambiante et le produit de réaction est séché à des températures comprises entre 35 °C et 75 °C, de préférence sous vide.

7. Procédé selon l'une quelconque des revendications 1 à 6, **caractérisé en ce que** le solvant est le toluène.

8. Procédé selon l'une quelconque des revendications 1 à 7, **caractérisé en ce qu'**on utilise de 1 à 2 équivalents, en particulier de 1,5 à 1,7 équivalent, de façon particulièrement préférée 1,6 équivalent de chlorure de thionyle, par rapport au composé de formule (II).

9. Procédé selon l'une quelconque des revendications 1 à 8, **caractérisé en ce que** la réaction avec le chlorure de thionyle s'effectue à une température de 30 à 50 °C, de préférence de 35 à 45 °C, pendant 1 à 4 heures, de préférence 2 à 3 heures.

10. Procédé selon l'une quelconque des revendications 4 à 9, **caractérisé en ce que** le composé (II) présente la configuration (IIa) et/ou (IIb) :

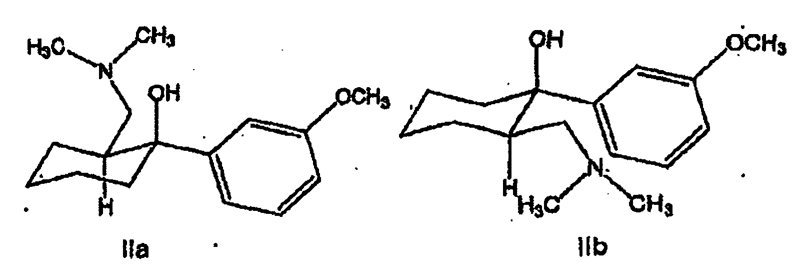

11. Procédé selon l'une quelconque des revendications 4 à 10, **caractérisé en ce que** le composé (II) est utilisé sous forme d'énantiomère ayant la configuration absolue (IIa) :

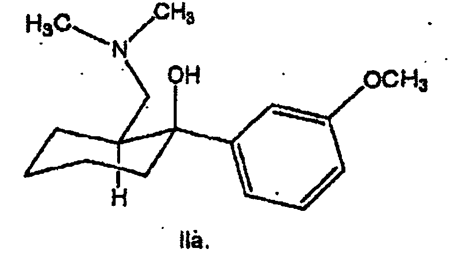

12. Procédé selon l'une quelconque des revendications 4 à 11, pour la préparation d'un composé de formule générale (III) 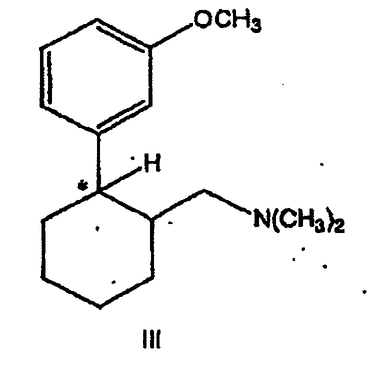 sous forme de ses racémates, de ses stéréoisomères purs, en particulier énantiomères ou diastéréoisomères, ou sous forme de mélanges des stéréoisomères, en particulier des énantiomères ou diastéréoisomères, en un rapport quelconque de mélange ;
sous la forme représentée ou sous forme de ses sels, en particulier des sels physiologiquement acceptables, de façon particulièrement préférée sous forme du chlorhydrate, et/ou sous forme de ses produits de solvatation, en particulier des hydrates ;
**caractérisé en ce qu'**on soumet le composé de formule (I) en une étape
(d) à une hydrogénation catalytique avec du palladium-sur-charbon actif en tant que catalyseur.

13. Procédé selon la revendication 12, **caractérisé en ce que** l'hydrogénation (d) s'effectue avec conservation de la configuration stéréochimique au niveau de l'atome de carbone désigné par un astérisque (*) dans la formule (I) ou (III).

14. Procédé selon l'une quelconque des revendications 12 ou 13, pour la préparation d'un composé (IV) 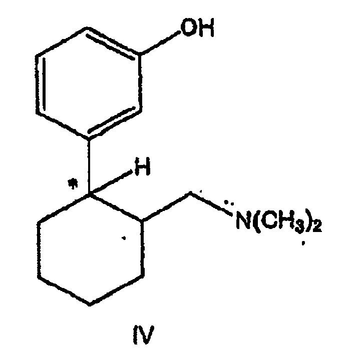 sous forme de ses racémates, de ses stéréoisomères purs, en particulier énantiomères ou diastéréoisomères, ou sous forme de mélanges des stéréoisomères, en particulier des énantiomères ou diastéréoisomères, en un rapport quelconque de mélange ;
sous la forme représentée ou sous forme de ses sels, en particulier des sels physiologiquement acceptables, de façon particulièrement préférée sous forme du chlorhydrate, et/ou sous forme de ses produits de solvatation, en particulier des hydrates ;
**caractérisé en ce qu'**on soumet le composé (III), sous forme de ses racémates, de ses stéréoisomères purs, en particulier énantiomères ou diastéréoisomères, ou sous forme de mélanges des stéréoisomères, en particulier des énantiomères ou diastéréoisomères, en un rapport quelconque de mélange ;
sous la forme représentée ou sous forme de ses sels, en particulier des sels physiologiquement acceptables, de façon particulièrement préférée sous forme du chlorhydrate, et/ou sous forme de ses produits de solvatation, en particulier des hydrates ;
en une étape
(e) à une coupure d'éther méthylique avec de la méthionine/de l'acide méthanesulfonique.

15. Procédé selon la revendication 14, **caractérisé en ce qu'**à partir du composé ayant la configuration absolue (IIa) 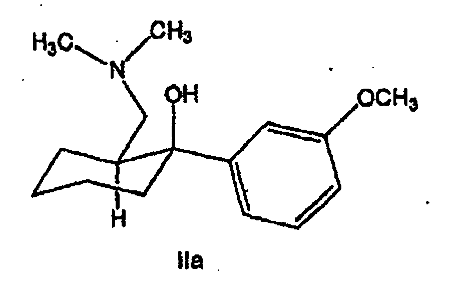 on prépare, par exécution des étapes (a) à (e) du procédé, le composé ayant la configuration absolue (IVa) 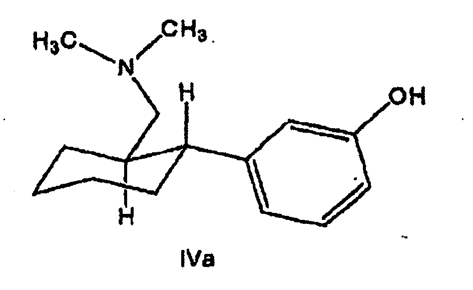 sous forme de son chlorhydrate et/ou de son chlorhydrate dihydrate.
